(19) Europäisches Patentamt
European Patent Office
Office européen des brevets

(11) Publication number: 0 466 573 B1

# (12) EUROPEAN PATENT SPECIFICATION

(45) Date of publication of patent specification: **15.02.95**

(51) Int. Cl.$^6$: **C09K 19/20**, C07C 43/00

(21) Application number: **91401900.5**

(22) Date of filing: **09.07.91**

## (54) Liquid crystal compositions.

(30) Priority: **13.07.90 JP 185740/90**

(43) Date of publication of application:
**15.01.92 Bulletin 92/03**

(45) Publication of the grant of the patent:
**15.02.95 Bulletin 95/07**

(84) Designated Contracting States:
**DE FR GB**

(56) References cited:
**EP-A- 0 422 613**

**PATENT ABSTRACTS OF JAPAN vol. 11, no. 206 (C-433)(2653) 3 July 1987**

**LIOUID CRYSTALS vol. 2, no. 1, 1987, pages 63 - 71; D. COATES: '"the influenceof alkyl chain branching on smectic C formation"'**

**LIOUID CRYSTALS vol. 6, no. 2, 1989, pages 167 - 174; Y. SUZUKI ET AL.: '"new flourine containing ferroelectric liquid crystal compounds showing tristable switching, page 168 par. 2**

(73) Proprietor: **Showa Shell Sekiyu Kabushiki Kaisha**
**2-5, Kasumigaseki 3-chome**
**Chiyoda-ku**
**Tokyo (JP)**

(72) Inventor: **Isozaki, Tadaaki, c/o Showa Shell Sekiyu K.K.**
**2-5, Kasumigaseki 3-chome**
**Chiyoda-ku,**
**Tokyo 100 (JP)**
Inventor: **Mogamiya, Hiroyuki, c/o Showa Shell Sekiyu K.K.**
**2-5, Kasumigaseki 3-chome**
**Chiyoda-ku,**
**Tokyo 100 (JP)**
Inventor: **Aihara, Yoshihiko, c/o Showa Shell Sekiyu K.K.**
**2-5, Kasumigaseki 3-chome**
**Chiyoda-ku,**
**Tokyo 100 (JP)**
Inventor: **Hagiwara, Takashi, c/o Showa Shell Sekiyu K.K.**
**2-5, Kasumigaseki 3-chome**
**Chiyoda-ku,**
**Tokyo 100 (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 466 573 B1

(74) Representative: **Bourgognon, Jean-Marie et al**
**Cabinet Flechner**
**22, Avenue de Friedland**
**F-75008 Paris (FR)**

## Description

The present invention is to provide a liquid crystal composition containing an antiferroelectric liquid crystal compound, and the present liquid crystal composition is used for display devices or electrooptical devices which take advantage of the response to electric field.

Furthermore, the present invention relates to an antiferroelectric liquid crystal composition exhibiting tristable molecular alignments.

As electrooptical devices comprising liquid crystals, electrooptical devices comprising nematic liquid crystals such as those of DSM, TN, G-H or STN types have been developed and put to practical use. These electrooptical devices comprising the nematic liquid crystals have a defect of a very slow respose in the range from several milliseconds to several ten milliseconds and thus are limited in their applications. The slow response of devices utilizing a nematic liquid crystal is attributed to the fact that the torque for moving molecules is based on the anisotropy of dielectric constant and thus the power is not strong. Among these backgrounds, a ferroelectric liquid crystal which exhibits spontaneous polarization (Ps) and a strong torque based on Ps x E (E: applied electric field) and is capable of a high speed response in the range of several μsec to several ten μsec has been developed by Meyer et al. (Le Journal de Physique, 36, 1975, L-69). Furthermore, there is disclosed a ferroelectric liquid crystal in Japanese Patent Laid-Open Publication No. 307837/1988, there have already been proposed several high speed electrooptical devices comprising ferroelectric liquid crystals.

A typical example includes a device in which the helical structure is released by the force of wall faces and the two molecular alignments parallel to the wall faces is changed by the polarity of an applied electric field (see, for example, Japanese Patent Laid-Open Publication No. 107216/1981).

The aforementioned device is composed on the assumption of the presence of a compound which exhibits such an ideal bistable states as is shown by a field response wave pattern in Fig. 1. However, no compound which exhibits such an ideal bistable states as described above has been found, and bistable liquid crystals synthesized hitherto show a field response wave pattern in Fig. 2 but not field response wave pattern in Fig. 1. It is the present state that if a device which exhibits a response wave pattern as shown in Fig. 2 is intended to be used for a switching circuit of light, such a pattern has a profile that transmittance varies gradually with the variation of an applied voltage from the minus side to the plus side and thus the object cannot be accomplished sufficiently with such a simple change of applied voltage as "on" and "off". Moreover, a bistable state liquid crystal having been synthesized is hard to form a monodomain structure as an ideal molecular alignment in the stage of a S*c phase at no electric field, and it causes disclination (defect) or twist which is the disturbance of the molecular alignment. It is thus difficult to realize the aforementioned ideal bistable alignment in a large area. Furthermore, it has a low threshold value (voltage at which the brightness varies at a predetermined extent), so that the dynamic drive of it may cause the lowering of contrast or the decrease of the range of viewing angle. The bistable state liquid crystal hitherto synthesized has no memory effect, since it cannot exhibit a hysteresis as shown in Fig. 1 but exhibits only a hysteresis as shown in Fig. 2. Thus, it is necessary to impress continuously a voltage at $v_3$ in Fig. 2 or to apply a high frequency in order to maintain a stable response in the S*c phase in the liquid crystal, and it cannot avoid a large energy loss.

Eventually, it is the present state that many a problems remain unsolved in conventional ferroelectric liquid crystal electrooptical devices notwithstanding the earnest desire of a high speed liquid crystal electrooptical device which takes advantage effectively of an applied field and a bond having a strong molecular alignment obtained in a ferroelectric liquid crystal.

Thus, there have been conducted researches on liquid crystal compounds exhibiting tristable states, characterized in that the liquid crystal compound realizes a stable molecular alignment having a distinct light-dark contrast depending on electric field applied, generates a distinct threshold property and a distinct hysteresis as shown in Fig. 3, realizes easily dynamic drive and is capable of a high speed response.

As a result, there have been proposed liquid crystal compounds having tristable states as disclosed in Japanese Patent Laid-Open Publications Nos. 316367/1989, 316372/1989 and 28128/1990.

However, the liquid crystal compounds represented by the formulae:

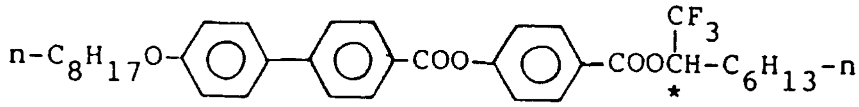

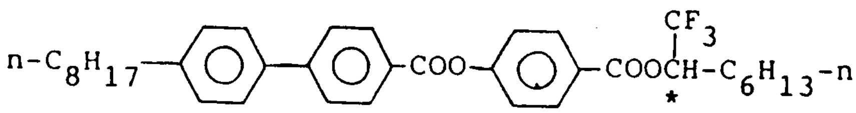

which are liquid crystal compounds exhibiting tristable states, have defects in their preparation processes that they are required for optical resolution and thus result in the expensive preparation costs because they are asymmetric compounds. As one of the methods for improving the defects without diminishing the tristable states, there is a method for dilution and extending by adding other organic compounds. There is also another problem in this case that the tristable states are diminished by the addition of the other organic compounds in an amount of 20% or more, in some cases 10% or more.

The object of the present invention is to provide a liquid crystal compound which is useful as a diluent of liquid crystal compounds exhibiting tristable states and exhibits per se a smectic phase.

Another object of the present invention is to provide a liquid crystal, composition which employs a novel diluent and exhibits tristable states.

Fig. 1 shows the hysteresis of an ideal bistable state liquid crystal which has not in fact been obtained,
Fig. 2 shows the hysteresis of a practical bistable state liquid crystal having been hitherto synthesized,
Fig. 3 shows the hysteresis of a tristable state liquid crystal composition according to the present invention, respectively, in which the abscissa represents applied voltage and the ordinate represents transmittance (%), and
Fig. 4 shows the infrared spectrum (KBr) of the compound of Reference Example.

The present invention relates to the use as diluent of a liquid crystal compound exhibiting a smectic phase represented by the formula

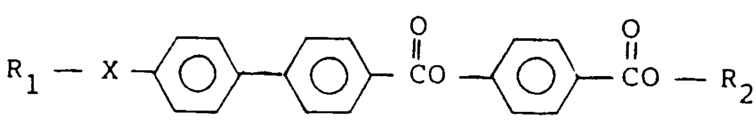 [I]

wherein $R_1$ represents an alkyl group having 3 - 20 carbon atoms,
$R_2$ represents an alkyl group having 3 - 20 carbon atoms, and
X represents a group

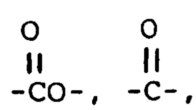

-O- or a single bond.

A compound of this kind is disclosed in LIQUID CRYSTALS vol. 2, no. 1 1987, p. 68 table 6.

The present invention relates to a liquid crystal composition in which the liquid crystal compound represented by the formula [I] and the other liquid crystal compounds are also incorporated.

The present invention further relates to providing a liquid crystal composition which employs a novel diluent represented by the formula [I] and exhibits tristable states.

The liquid crystal compound used according to the present invention preferably includes the compounds represented by the formulae:

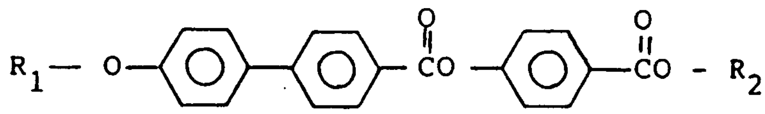

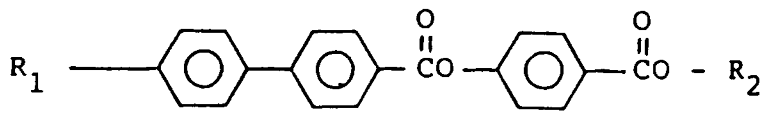

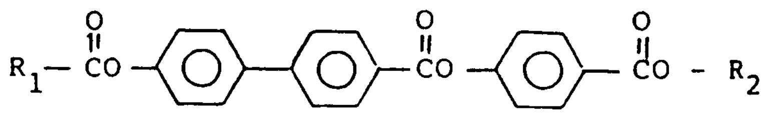

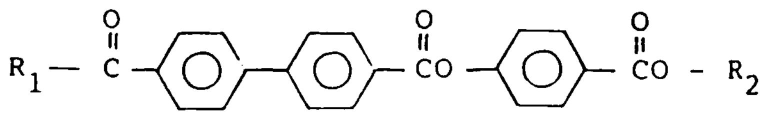

wherein $R_1$ and $R_2$ each represents alkyl group having 3 - 20 carbon atoms.

Particularly, the liquid crystal compound used according to the present invention is required to have such a skeleton structure as

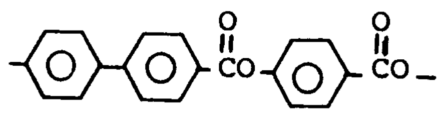 .

As the typical synthesis of the compounds used according to the present invention, there are mentioned the methods as follows:

(1) 4-Hydroxybenzoic acid and an alkyl alcohol are subjected to an esterification reaction in the presence of sulfuric acid as a catalyst to give an alkyl 4-hydroxybenzoate. A 4'-alkyloxy-4-carboxybiphenyl is reacted with thionyl chloride to give a 4'-alkyloxybiphenyl-4-carboxylic acid chloride. The alkyl 4-hydroxybenzoate and the 4'-alkyloxybiphenyl-4-carboxylic acid chloride were allowed to react to give a 4-alkyloxycarbonylphenyl 4'-alkyloxybiphenyl-4-carboxylate; and

(2) 4-Hydroxybenzoic acid and an alkyl alcohol are subjected to an esterification reaction in the presence of sulfuric acid as a catalyst to give an alkyl 4-hydroxybenzoate. A 4'-alkyl-4-carboxybiphenyl is reacted with thionyl chloride to give a 4'-alkyloxybiphenyl-4-carboxylic acid chloride. The alkyl 4-hydroxybenzoate and the 4'-alkyloxybiphenyl-4-carboxylic acid chloride were allowed to react to give a 4-alkyloxycarbonylphenyl 4'-alkylbiphenyl-4-carboxylate.

The compounds used in the present invention are described with reference to examples without limitation thereto.

Example 1

1) Synthesis of n-nonyl 4-hydroxybenzoate

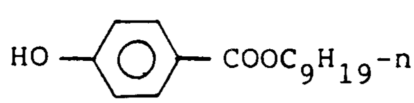

4-Hydroxybenzoyl chloride (5.52 g) was dissolved in 10 ml of dichloroethane, and a solution of nonyl alcohol (6.34 g) in 95 ml of dichloroethane was added. Several drops of concentrated sulfuric acid were added, and the mixture was heated under reflux for about 12 hours. The reaction mixture was poured into water, and the organic layer was separated, washed with water, a dilute aqueous $Na_2CO_3$ solution and water in this sequence and dried over anhydrous magnesium sulfate. The solvent was removed by distillation to give 7.8 g of the desired product.

2) Synthesis of 4-n-nonyloxycarboxyphenyl 4′-n-decyloxybiphenyl-4-carboxylate

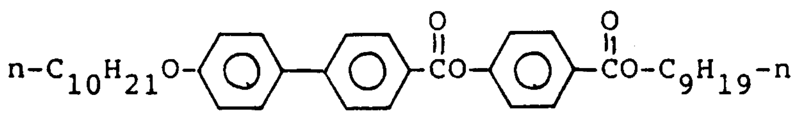

4′-n-Decyloxy-4-carboxybiphenyl (3.00 g) was heated under reflux for 6 hours together with an excessive amount of thionyl chloride, and the unaltered thionyl chloride was removed by distillation to give about 3 g of 4′-n-decyloxybiphenyl-4-carboxylic acid chloride.

The n-nonyl 4-hydroxybenzoate synthesized in the step 1) (1.40 g) and triethylamine (0.56 g) were dissolved in 30 ml of methylene chloride. The solution of the chloride synthesized above (2.18 g) in 35 ml of methylene chloride was added dropwise to the aforementioned solution at room temperature. Dimethylaminopyridine (0.19 g) was dissolved in 5 ml of methylene chloride and added further to the solution. The resulting mixture was heated under reflux for about 12 hours and then poured into water. Extraction was conducted with methylene chloride, and the organic layer was washed with water, an aqueous sodium carbonate solution and water in this sequence and dried over anhydrous magnesium sulfate. The solvent was removed by distillation, and the residue was purified by column chromatography (hexane: ethyl acetate = 10 : 0.5) to give 1.07 g of the desired product.

The result of the NMR spectrographical analysis of the desired product are shown in Table 1.

Table 1

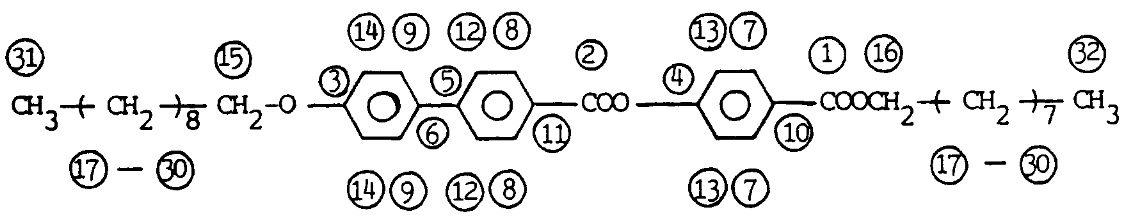

| CARBON | PPM | REMARKS |
|---|---|---|
| 1 | 165.9 | carbon of ester carbonyl |
| 2 | 164.6 | carbon of ester carbonyl |
| 3 | 159.6 | aromatic ring carbon adjacent to oxygen |

Table 1 (continued)

| CARBON | PPM | REMARKS |
|---|---|---|
| 4 | 154.6 | aromatic ring carbon adjacent to ester oxygen |
| 5 | 146.2 | aromatic ring carbon adjacent to aromtic ring |
| 6 | 131.8 | aromatic ring carbon adjacent to aromatic ring |
| 7 | 131.1 | aromatic ring carbon (for two atoms) |
| 8 | 130.8 | aromatic ring carbon (for two atoms) |
| 9 | 128.4 | aromatic ring carbon (for two atoms) |
| 10 | 128.1 | aromatic ring carbon adjacent to ester carbon |
| 11 | 127.0 | aromatic ring carbon adjacent to ester carbon |
| 12 | 126.6 | aromatic ring carbon (for two atoms) |
| 13 | 121.7 | aromatic ring carbon (for two atoms) |
| 14 | 115.0 | aromatic ring carbon (for two atoms) |
| 15 | 68.1 | methylene carbon adjacent to oxygen |
| 16 | 65.3 | methylene carbon adjacent to ester oxygen |
| 17 | 31.9 | methylene carbon |
| 18 | 31.8 | methylene carbon |
| 19 | 29.6 | methylene carbon |
| 20 | 29.5 | methylene carbon |
| 21 | 29.5 | methylene carbon |
| 22 | 29.4 | methylene carbon |
| 23 | 29.3 | methylene carbon |
| 24 | 29.3 | methylene carbon |
| 25 | 29.2 | methylene carbon |
| 26 | 28.7 | methylene carbon |
| 27 | 26.0 | methylene carbon |
| 28 | 26.0 | methylene carbon |
| 29 | 22.7 | methylene carbon |

Table 1 (continued)

| CARBON | PPM | REMARKS |
|---|---|---|
| 30 | 22.6 | methylene carbon |
| 31 | 14.1 | terminal methyl carbon |
| 32 | 14.1 | terminal methyl carbon |

3) The compound synthesized in Example 1 represented by the formula (A)

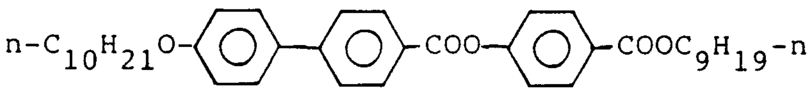

(A)

showed the following phase transition temperatures (°C) on the observation with a polarizing microscope equipped with a hot stage:

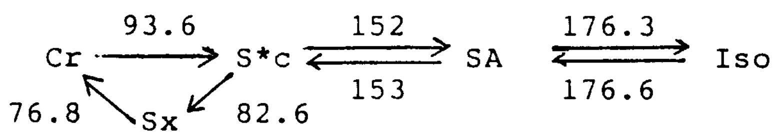


wherein

Iso: isotropic phase,
SA: smectic A phase,
S*c: smectic C phase,
Sx: smectic phase in a high order, and
Cr: crystal phase.

Example 2

4-n-Nonyloxycarbonylphenyl 4′-n-decylbiphenyl-4-carboxylate

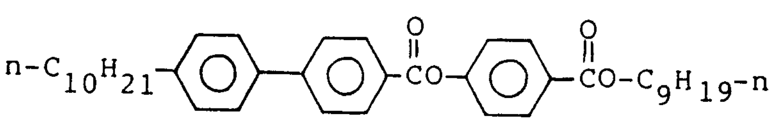

After 4′-n-decyl-4-carboxybiphenyl (3.10 g) was heated under reflux for 6 hours together with an excessive amount of thionyl chloride, the unaltered thionyl chloride was removed by distillation to give about 3 g of 4′-n-decylbiphenyl-4-carboxylicacid chloride.

The n-nonyl 4-hydroxybenzoate (1.40 g) synthesized in the step 1) of Example 1 and triethylamine (0.56 g) were dissolved in 30 ml of methylene chloride. The solution of the chloride synthesized above (2.10 g) in 35 ml of methylene chloride was added dropwise to the aforementioned solution at room temperature. Dimethylaminopyridine (0.19 g) was dissolved in 5 ml of methylene chloride and added further to the solution. The resulting mixture was heated under reflux for about 12 hours and then poured into water. Extraction was conducted with methylene chloride, and the organic layer was washed with water, an aqueous sodium carbonate solution and water in this sequence and dried over anhydrous magnesium sulfate. The solvent was removed by distillation, and the residue was purified by column chromatography (hexane: ethyl acetate = 10 : 0.5) to give 1.07 g of the desired compound.

The results of the NMR spectrographical analysis of the above compound are shown in Table 2.

Table 2

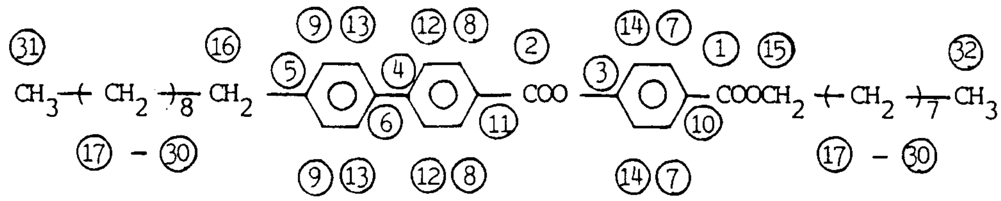

| CARBON | PPM | REMARKS |
|---|---|---|
| 1 | 165.9 | carbon of ester carbonyl |
| 2 | 164.5 | carbon of ester carbonyl |
| 3 | 154.6 | aromatic ring carbon adjacent to ester oxygen |
| 4 | 146.5 | aromatic ring carbon adjacent to aromatic ring |
| 5 | 143.5 | aromatic ring carbon adjacent to methylene carbon |
| 6 | 136.9 | aromatic ring carbon adjacent to aromatic ring |
| 7 | 131.1 | aromatic ring carbon (for two atoms) |
| 8 | 130.7 | aromatic ring carbon (for two atoms) |
| 9 | 129.0 | aromatic ring carbon (for two atoms) |
| 10 | 128.1 | aromatic ring carbon adjacent to ester carbon |
| 11 | 127.4 | aromtic ring carbon adjacent to ester carbon |
| 12 | 127.1 | aromatic ring carbon (for two atoms) |
| 13 | 127.0 | aromatic ring carbon (for two atoms) |
| 14 | 121.7 | aromatic ring carbon (for two atoms) |
| 15 | 65.2 | methylene carbon adjacent to ester oxygen |
| 16 | 35.6 | methylene carbon adjacent to aromatic ring |
| 17 | 31.9 | methylene carbon |

Table 2 (continued)

| CARBON | PPM | REMARKS |
|---|---|---|
| 18 | 31.8 | methylene carbon |
| 19 | 31.4 | methylene carbon |
| 20 | 29.6 | methylene carbon |
| 21 | 29.6 | methylene carbon |
| 22 | 29.5 | methylene carbon |
| 23 | 29.5 | methylene carbon |
| 24 | 29.3 | methylene carbon |
| 25 | 29.3 | methylene carbon |
| 26 | 29.2 | methylene carbon |
| 27 | 28.7 | methylene carbon |
| 28 | 26.9 | methylene carbon |
| 29 | 22.7 | methylene carbon |
| 30 | 22.6 | methylene carbon |
| 31 | 14.1 | terminal methyl carbon |
| 32 | 14.1 | terminal methyl carbon |

4) The compond synthesized in Example 2 represented by the formula:

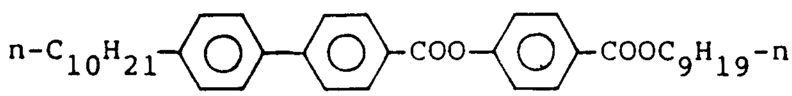 (B)

showed the following phase transition temperatures on the observation with a polarization microscope equipped with a hot stage:

$$Cr \xleftarrow{73.5} S^{*}c \xleftarrow{114} SA \xleftarrow{148.2} Iso$$

wherein

Iso : isotropic phase,
SA : smectic A phase,
S*c : smectic C phase, and
Cr : crystal phase.

Reference Examples (Synthesis of liquid crystal compounds exhibiting tristable states)

1) Synthesis of 1,1,1-trifluoro-2-octyl 4-benzyloxybenzoate

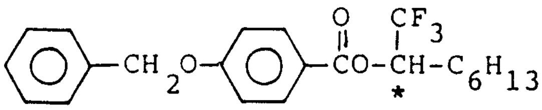

After 4-benzyloxybenzoic acid chloride (4.3 g) was dissolved in 50 ml of methylene chloride, a solution of an optically active 1,1,1-trifluoro-2-octanol (2.9 g), dimethylamino pyridine (0.6 g) and triethylamine (1.7 g) in 50 ml of methylene chloride was added portionwise under cooling.

The reaction mixture was allowed to raise the temperature to room temperature and subjected to reaction for a day. The reaction mixture was poured into ice-water, and extraction was conducted with methylene chloride. The methylene chloride layer was washed with a dilute hydrochloric acid, water, a 1N aqueous sodium carbonate solution and water in this sequence, and the organic layer was dried over anhydrous magnesium sulfate. The solvent was removed by distillation to give a crude product, which was purified by column chromatography on silica gel with toluene as an eluent and further recrystallized from ethanol to give 3.8 g of the desired compound.

2) Synthesis of 1,1,1-trifluoro-2-octyl 4-hydroxybenzoate

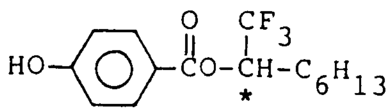

The compound obtained in the step 1) was dissolved in 100 ml of methanol, 0.4 g of a 10% Pd on carbon was added, and hydrogenolysis was conducted under the atmosphere of hydrogen to give 2.8 g of the desired compound.

3) Synthesis of 4-(1,1,1-trifluoro-2-octyloxycarbonyl)phenyl 4′-n-octyloxybiphenyl-4-carboxylate

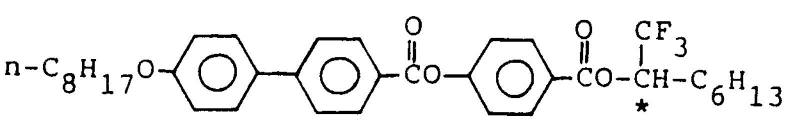

After 4-n-octyloxybiphenyl-4′-carboxylic acid (3.0 g) was heated under reflux together with an excessive amount of thionyl chloride for 6 hours, the unaltered thionyl chloride was removed by distillation to give 4-n-octyloxybiphenyl-4′-carboxylic acid chloride.

To a solution of the acid chloride in 50 ml of methylene chloride was added slowly under ice cooling a solution of 1,1,1-trifluoro-2-octyl-4-hydroxybenzoate (2.8 g), triethylamine (1.0 g) and dimethylaminopyridine (0.3 g) in 50 ml of methylene chloride, and the mixture was allowed to react at room temperature for a day.

The reaction mixture was next poured into ice-water and extracted with methylene chloride. The methylene chloride layer was washed with a dilute hydrochloric acid, water, an aqueous sodium carbonate solution and water in this sequence and dried over anhydrous sodium sulfate. The solvent was removed by distillation to give a crude product, which was purified by column chromatography on silica gel with toluene as an eluent to give 2.1 g of the optically active desired compound.

For the measurement of the phase transition temperatures, the compound further purified by recrystallization from anhydrous ethanol was employed.

The infrared spectrum (KBr) of the desired product is shown in Fig. 4.

The compond synthesized in Reference Example represented by the formula;

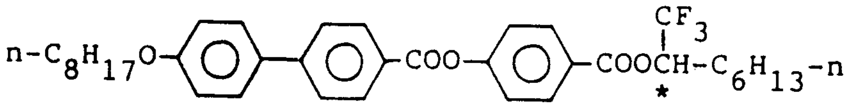

(C)

showed the following phase transition temperatures (°C) on the observation with a polarizing microscope equipped with a hot stage:

$$Cr \underset{74}{\overset{80}{\rightleftarrows}} S^{*}(3) \underset{115}{\rightleftarrows} SA \underset{123.4}{\overset{125.3}{\rightleftarrows}} Iso$$

wherein

- Iso : isotropic phase,
- SA : smectic A phase,
- S*(3) : phase exhibiting tristable states, and
- Cr : crystal phase.

Example 3

The substance comprising a mixture of equivalent amounts of compounds (A) and (C) showed the following phase transition temperatures (°C) on the observation with a polarizing microscope equipped with a hot stage:

$$Sx \underset{30}{\overset{60}{\rightleftarrows}} S^{*}I \underset{65}{\overset{71}{\rightleftarrows}} S^{*}(3) \underset{109.9}{\overset{110}{\rightleftarrows}} S^{*}c \underset{128}{\overset{128}{\rightleftarrows}} SA \underset{158.9}{\overset{159.7}{\rightleftarrows}} Iso$$

wherein

- Iso: isotropic phase,
- SA : smectic A phase,
- S*c : chiral smetic C phase,
- S*(3) : phase exhibiting tristable states, and
- S*I : chiral smectic I phase, and
- Sx : smectic phase in a high order.

Example 4

The substance comprising a mixture of equivalent amounts of compounds (B) and (C) showed the following phase transition temperatures (°C) on the observation with a polarizing microscope equipped with a hot stage:

$$Cr \underset{17}{\overset{68}{\rightleftarrows}} S^{*}(3) \underset{85}{\overset{86}{\rightleftarrows}} S^{*}c \underset{114}{\overset{115}{\rightleftarrows}} SA \underset{140.4}{\overset{140}{\rightleftarrows}} Iso$$

wherein

- Iso: isotropic phase,
- SA: smectic A phase,

S*c: chiral smectic C phase,
S*(3): phase exhibiting tristable states, and
Cr: crystal phase.

According to the present invention, a use of a liquid crystal compound is provided, and the cost for preparing a tristable antiferroelectric liquid crystal composition can be reduced by adding the liquid crystal compound to the tristable antiferroelectric liquid crystal composition.

## Claims

**1.** The use of a liquid crystal compounds represented by the formula (I):

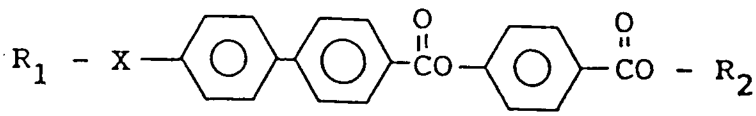

I

wherein $R_1$ represents an alkyl group having 3-20 carbon atoms,
$R_2$ represents a straight chain alkyl group having 3-20 carbon atoms,
X represents a group

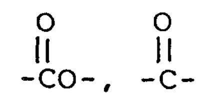

or -O-, or a
single bond, as diluents for liquid crystal compounds which exhibit S* (3) phase and optically tristable states.

**2.** The use as claimed in claim 1, wherein the liquid crystal compounds which exhibit S* (3) phase and optically tristable states are represented by the formulae

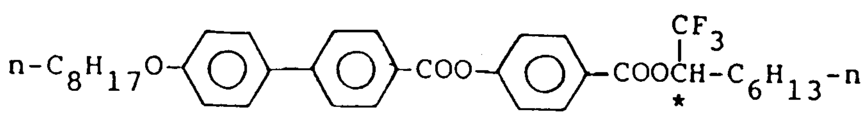

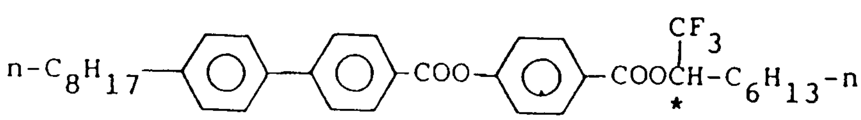

## Patentansprüche

**1.** Verwendung von Flüssigkristall-Verbindungen, die durch die Formel (I) wiedergegeben sind:

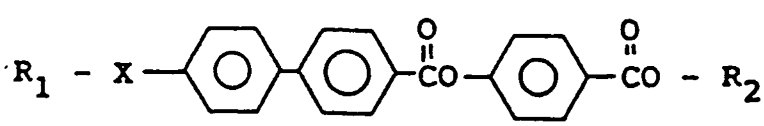

(I)

worin
$R_1$ für eine Alkylgruppe mit 3 bis 20 Kohlenstoffatomen steht;
$R_2$ für eine geradkettige Alkylgruppe mit 3 bis 20 Kohlenstoffatomen steht;
X für eine Gruppe

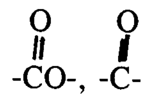

oder -O- oder eine Einfachbindung steht, als Verdünnungsmittel für Flüssigkristall-Verbindungen, die eine S*(3)-Phase und optisch tristabile Zustände zeigen.

**2.** Verwendung nach Anspruch 1, worin die Flüssigkristall-Verbindungen, die eine S*(3)-Phase und optisch tristabile Zustände Zeigen, wiedergegeben werden durch die Formeln

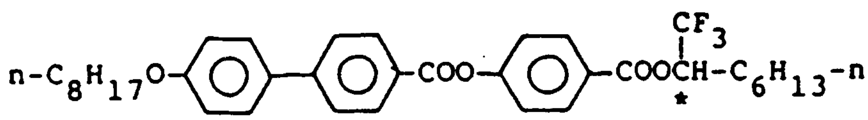

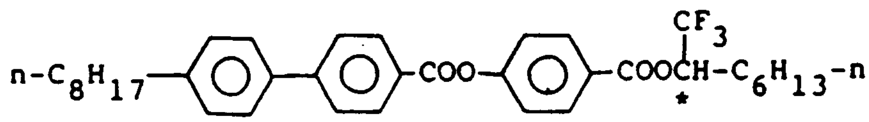

## Revendications

**1.** L'utilisation de cristaux liquides de formule (I):

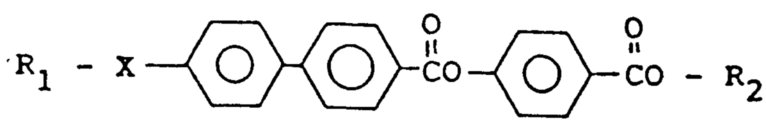
I

dans laquelle $R_1$ représente un groupe alcoyle ayant de 3 à 20 atomes de carbone,
$R_2$ représente un groupe alcoyle linéaire de 3 à 20 atomes de carbone,
X représente un groupe

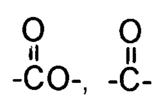

ou -O-, ou une liaison simple, comme diluant de cristaux liquides qui présentent une phase S*(3) et des états tristables optiquement.

**2.** L'utilisation suivant la revendication 1 dans laquelle les cristaux liquides qui présentent une phase S*(3) et des états tristables optiquement sont représentés par les formules :

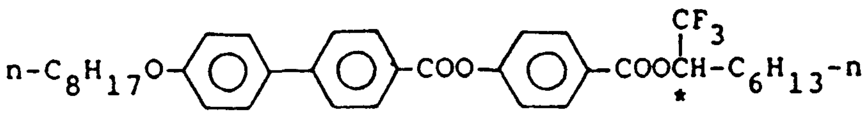

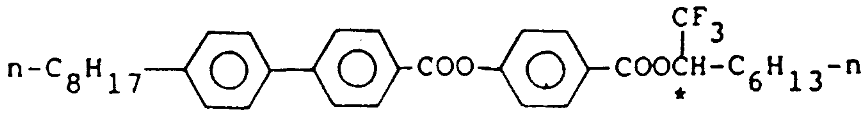

FIG. 1

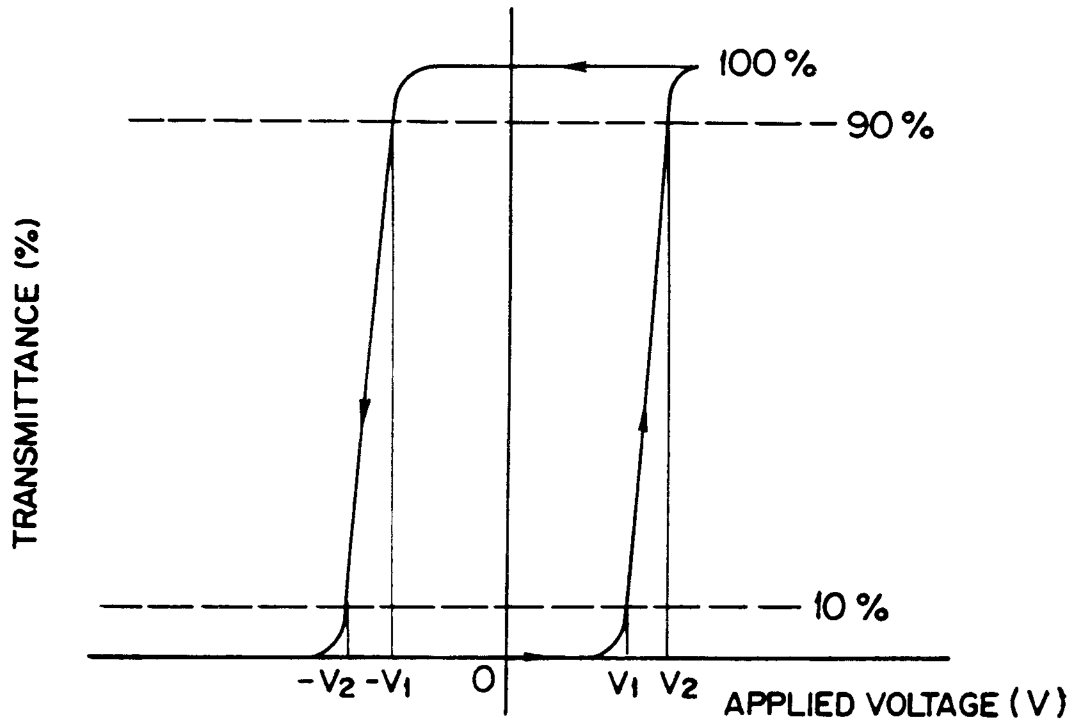
100 %
90 %
10 %
TRANSMITTANCE (%)
-V2 -V1
O
V1 V2
APPLIED VOLTAGE ( V )

FIG. 2

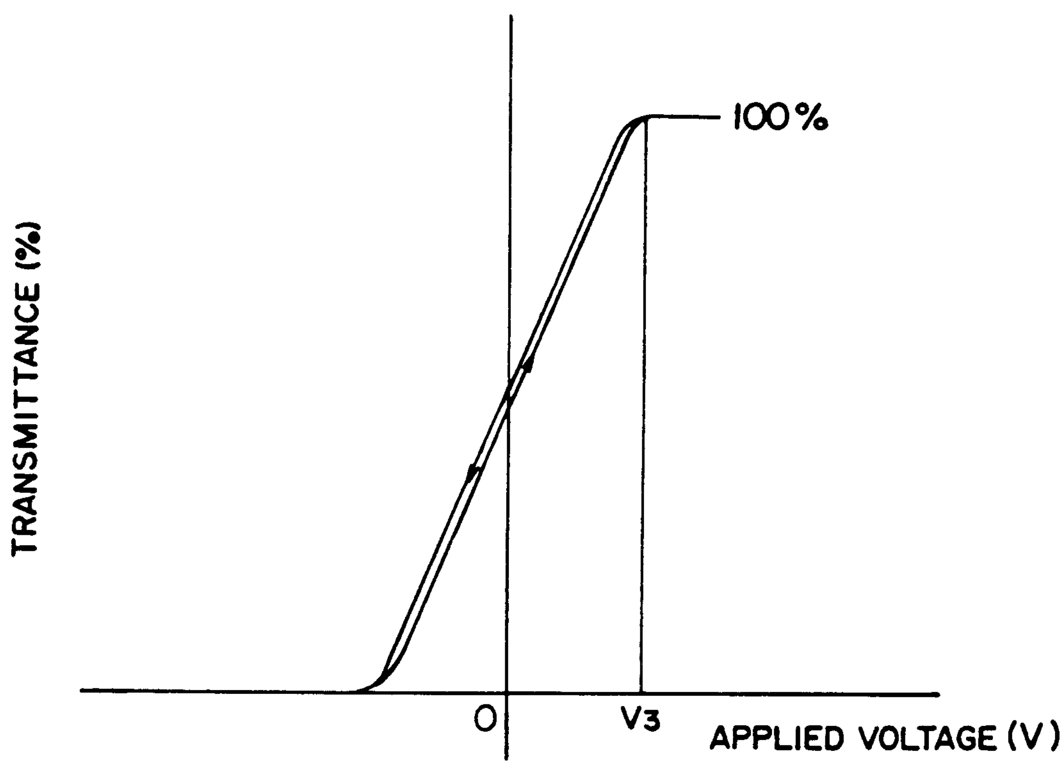
100%
TRANSMITTANCE (%)
O
V3
APPLIED VOLTAGE (V)

FIG. 3

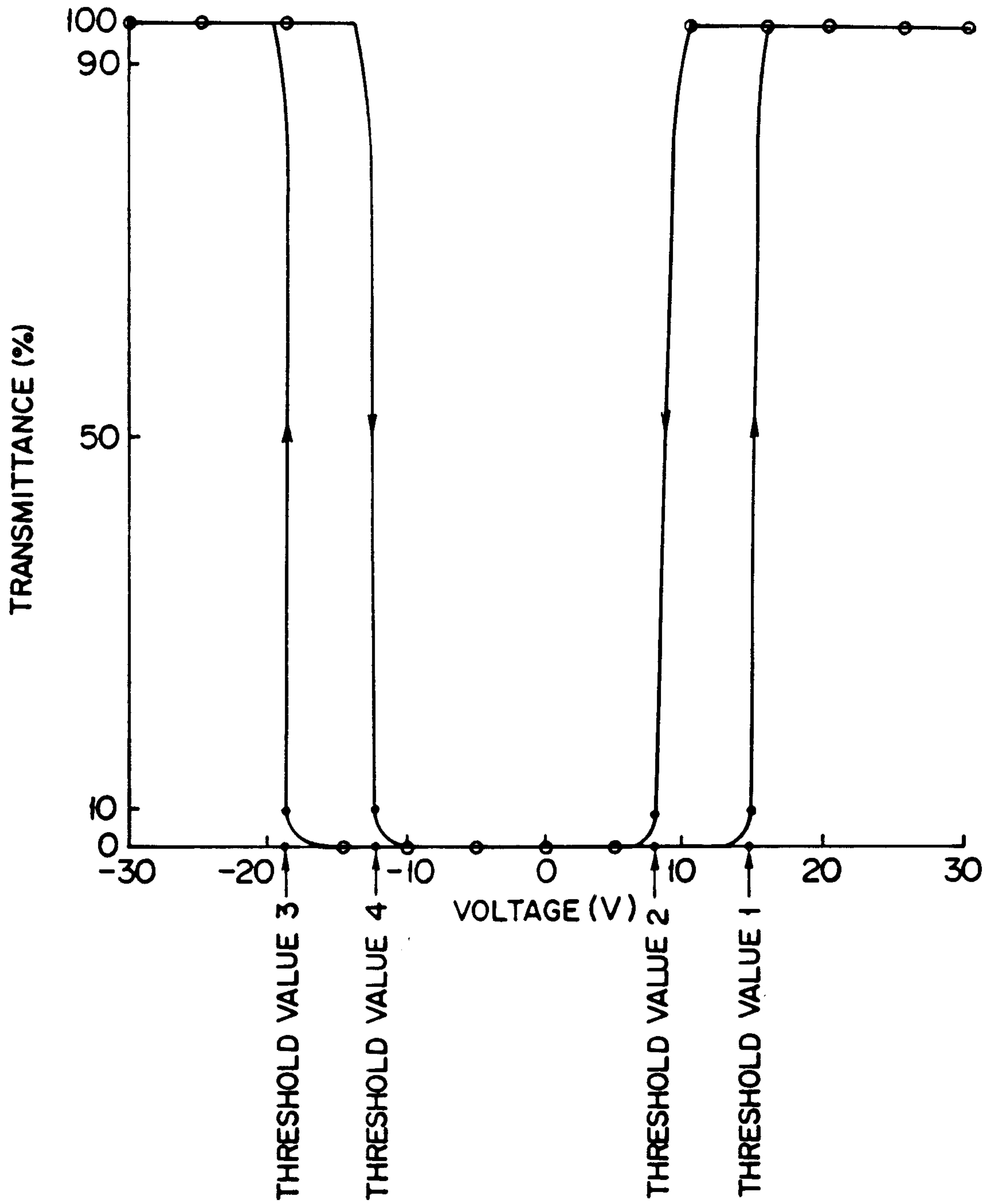
TRANSMITTANCE (%)
100
90
50
10
0
-30
-20
-10
0
10
20
30
VOLTAGE (V)
THRESHOLD VALUE 3
THRESHOLD VALUE 4
THRESHOLD VALUE 2
THRESHOLD VALUE 1

FIG. 4

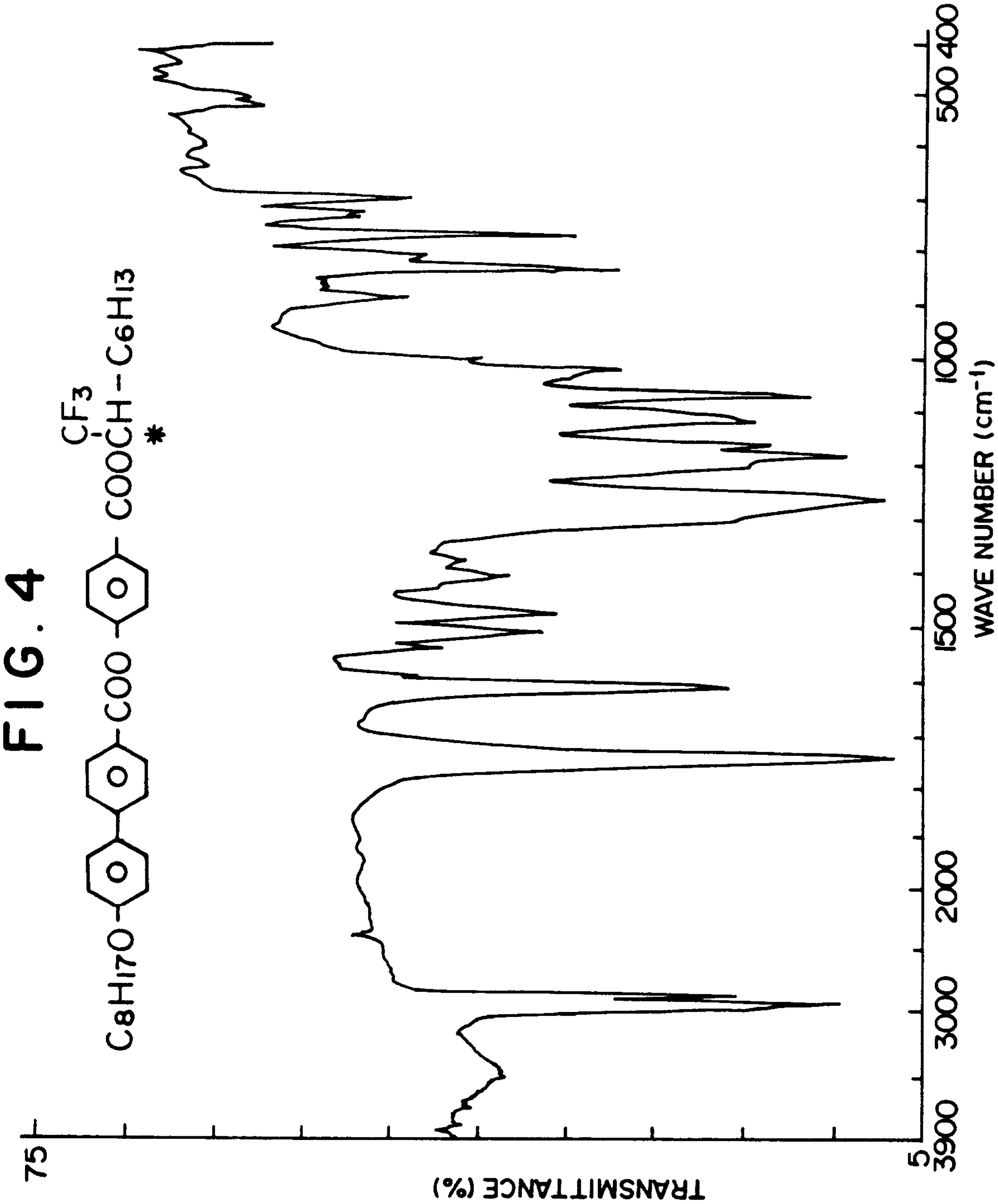
TRANSMITTANCE (%)
75
5
WAVE NUMBER (cm⁻¹)
3900
3000
2000
1500
1000
500
400